# EUROPÄISCHE PATENTSCHRIFT

(11) **EP 2 083 881 B1**
(45) Veröffentlichungstag und Bekanntmachung des Hinweises auf die Patenterteilung: **14.12.2016**
(21) Anmeldenummer: 07872073.7
(22) Anmeldetag: 22.10.2007
(51) Int. Cl.: A61M 1/16, A61M 1/34, A61J 1/10

(54) **HÄMODIALYSEGERÄT, HÄMODIAFILTRATIONSGERÄT, VERFAHREN ZUR PROBENNAHME BEI SOLCHEN GERÄTEN UND PROBENENTNAHMESET ZUR ANWENDUNG BEI SOLCHEN GERÄTEN UND VERFAHREN**
HEMODIALYSIS DEVICE, HEMODIAFILTRATION DEVICE, METHOD FOR TAKING A SAMPLE IN CORRESPONDING DEVICES AND SAMPLING KIT FOR USE IN CORRESPONDING DEVICES AND METHOD
APPAREIL D'HÉMODIALYSE, APPAREIL D'HÉMODIAFILTRATION, PROCÉDÉ DE PRÉLÈVEMENT D'ÉCHANTILLONS AU MOYEN DE TELS APPAREILS, ET KITS D'ÉCHANTILLONNAGE POUR L'UTILISATION DANS LESDITS APPAREILS, ET PROCÉDÉ CORRESPONDANT

(30) Priorität: 23.10.2006 DE 102006050272
(43) Veröffentlichungstag der Anmeldung: 05.08.2009
(73) Patentinhaber: Fresenius Medical Care Deutschland GmbH, 61352 Bad Homburg (DE)
(72) Erfinder: BALSCHAT, Klaus, 97525 Schwebheim (DE); SPICKERMANN, Reiner, 97535 Wasserlosen-Burghausen (DE); WINTER, Josef, 66346 Püttlingen (DE)
(74) Vertreter: Ziermann, Oliver
(86) Internationale Anmeldenummer: PCT/IB2007/004463
(87) Internationale Veröffentlichungsnummer: WO 2008/096202

(56) Entgegenhaltungen:
- EP-A- 0 711 569
- EP-A- 1 595 560
- DE-A1- 4 203 069
- FR-A- 2 696 644
- GB-A- 2 110 564
- US-A1- 2003 209 475

## Beschreibung

Die Erfindung betrifft das Gebiet von Geräten zur Nierenersatztherapie.

Bei der Hämodialyse wird Blut in einem extrakorporalen Kreislauf kontinuierlich einem Patienten entnommen, durch einen Hämodialysator geleitet und dem Patienten wieder reinfundiert. Dabei wird ein Stoffaustausch durchgeführt, der dem der Nieren ähnlich ist. Der Hämodialysator besteht aus zwei durch eine semipermeable Membran getrennte Kammern, von denen die eine vom Blut und die andere von einer Reinigungsflüssigkeit - der Dialysierflüssigkeit - durchflossen wird. Die handelsüblichen Hämodialysatoren weisen hierfür meist Tausende von Hohlfasern auf, deren Wände semipermeabel für die auszutauschenden Substanzen sind. Das Blut wird durch den Innenraum der Hohlfasern geleitet, während die Dialysierflüssigkeit in meist gegenläufiger Richtung in den Faserzwischenraum eingespeist und abgeführt wird.

Die Dialysierflüssigkeit weist Konzentrationen von Blutinhaltsstoffen wie Elektrolyten auf, die in etwa denen eines Gesunden entsprechen, damit die entsprechenden Konzentrationen im Blut auf einem normalen Niveau gehalten werden können. Aus dem Blut zu entfernende Stoffe wie zum Beispiel Kreatinin oder Harnstoff sind in der Dialysierflüssigkeit nicht enthalten, wodurch diese allein wegen des Konzentrationsgradienten an der Membran durch Diffusion aus dem Blut entfernt werden. Mit Hilfe eines Druckgradienten wird dem Blut überschüssiges Wasser durch Konvektion beziehungsweise Ultrafiltration entzogen.

Zur Steuerung derartiger Vorgänge werden Hämodialysegeräte eingesetzt, die zumeist auch die Zubereitung der Dialysierflüssigkeit aus Wasser und Konzentraten mit der richtigen Zusammensetzung und Temperatur sicherstellen. Diese Flüssigkeit wird in heutigen Hämodiafiltrationsgeräten auch dazu benutzt, eine durch erhöhte Konvektion herbeigeführte Blutreinigung (Hämofiltration) auszugleichen. Bei der Hämodiafiltration wird dem Blut des Patienten während einer Hämodialysebehandlung über den Hämodialysator eine größere Menge Ultrafiltrat entzogen, die bis auf die insgesamt zu entziehende Flüssigkeitsmenge wieder durch Substitutionsflüssigkeit ersetzt wird. Bei modernen Geräten insbesondere zur Behandlung des chronischen Nierenversagens wird hierfür die on-line während der Behandlung aufbereitete Dialysierflüssigkeit verwendet, indem eine von dem Dialysierflüssigkeitskreislauf abzweigende Leitung mit einem oder mehreren Filterstufen versehen und mit dem extrakorporalen Blutkreislauf stromauf und/oder stromab des Hämodialysators verbunden wird.

Die Dialysierflüssigkeit selbst wird aus Wasser und meist zwei Konzentraten gewonnen, die von dem Hämodialysegerät in einem vorgegebenen Verhältnis gemischt werden. Gleichzeitig wird die Dialysierflüssigkeit zur Durchführung einer Dialyse etwa auf Körpertemperatur erwärmt. Die Konzentrate können in kleineren Gebinden in flüssiger oder trockener Form vorliegen. In Kliniken ist auch der Einsatz von zentral versorgenden Ringleitungssystemen verbreitet.

Dabei müssen Wasser und Dialysierflüssigkeit nicht nur bei der Zusammensetzung und der Temperatur hohen Anforderungen genügen. Auch die mikrobiologische Reinheit ist sehr wichtig. Um die geforderten Standards bzgl. mikrobiologischer Anforderungen (Keime, Endotoxine) zu erfüllen und sicherzustellen, sind Kontrollen durch Probenentnahme durchzuführen. Die Probenentnahme ist komplex, weil Sekundärkontaminationen vermieden werden müssen, die die mikrobiologischen Untersuchungen verfälschen können.

Zur Durchführung der Probenentnahme werden bisher zusätzliche Komponenten eingesetzt. Verbreitet sind Entnahmestellen über selbstschließende Septen z.B. aus Silikon, die an einer durchflossenen Leitung eingebaut werden. Auch gibt es T-artige Entnahmestellen für Schlauchleitungsstücke, an die eine Spritze ohne Nadel angeschlossen werden kann. Bei allen diesen Lösungen ist die Einhaltung strenger Hygienevorschriften notwendig, um ein Einschleppen von Sekundärkontaminationen durch die Probenentnahme selbst zu verhindern.

Die DE 103 36 539 A1 beschreibt ein T-artiges Anschlussstück zur Probenentnahme von in Hämodialysegeräten verwendeten Flüssigkeiten, bei der das Septum einfach ausgewechselt werden kann. Die US 5,630,935 zeigt ein Probenentnahmeventil für die Wasserversorgungsleitung eines Hämodialysegerätes.

Die DE 28 38 414 C2 beschreibt ein Hämodialysegerät, bei dem mit Hilfe der Ultrafiltrationspumpe sowohl frische als auch verbrauchte Dialysierflüssigkeit an einen speziellen Probenport gefördert werden kann. Dies erfordert jedoch zusätzliche Leitungen sowie Anschlüsse, die außerdem regelmäßig in die Reinigungsprozedur miteinbezogen werden müssten.

Die EP 0 711 569 A1 beschreibt eine Einrichtung und ein Verfahren zur Ermittlung der Effektivität einer Hämodialysebehandlung und bestimmt dazu die bei einer Hämodialysebehandlung entfernten Urämietoxine. Dazu werden Dialysatproben genommen und in einem Probensammelbehälter gesammelt. In einer so genannten partiellen Dialysatsammlung wird über eine Ventilschaltung ein Teilstrom der Verbrauchten Dialysierflüssigkeit entnommen. Als bevorzugt wird eine Schalthäufigkeit (der Ventilschaltung) zwischen 30 und 100 pro Dialyse angegeben (Spalte 6, Zeile 39 - 41). Typischerweise werden etwa 2% der im Laufe der Dialysebehandlung verbrauchten Dilaysierflüssigkeit entnommen (Spalte 6, Zeile 24 -27). Die Steuerung der Ventile der Ventilschaltung und die Bemessung des Probeentnahmeintervalls kann bevorzugt auf einem gemessenen Fluss verbrauchter Dialyseflüssigkeit beruhen. So wird ein Ausführungsbeispiel eines Hämodialysegeräts mit Bilanzkammern offenbart bei dem ein Probennahmeventil synchron mit jeder n-ten Bilanzkammerumschaltung geschaltet wird. Diese Aspekte betreffen die Entnahme von Dialysat während einer Dialysebehandlung. Ein weiterer Aspekt der D1 betrifft eine besonders kontaminationsarme partielle Dialysatsammlung in einem Dialysegeröt, dass mit einem Rezirkulationsverfahren desinfiziert bzw. sterilisiert werden kann Eine konatminationsarme Dialysatsammlung wird dadurch erreicht, das ein Rezirkulationszweig des Hämodialysegeräts aufgetrennt wird. Die Sammlung von Dialysatproben kann so an einer aufgetrennten Stelle des Dialysatkreislaufs erfolgen. Der Rezirkulationskreislauf kann zur Desinfektion des Dialysegeräts genutzt werden, indem ein Desinfektionsmittelkonzentrat in den Rezirkulationskreislauf eingebracht wird.

Die GB 2 110 564 A offenbart eine extrakorporale Blutbehandlungsmaschine, mit schlauchförmigen Mittel und schlauchförmigen Abschnitten innerhalb einer Kassette, die so an die extrakorporale Blutbehandlungsmaschine gekoppelt werden kann, dass automatisch eine richtige Verbindung der schlauchförmigen Mittel an die Blutbehandlungsmaschine vorgenommen wird. Die Kassette für den Dialysatkreislauf weist einen Anschluss zur Entnahme von Proben auf.

In der US2003/0209475 A1 ist eine Hämodilaysemaschine mit einer berührungsempfindlichen Benutzerschnittstelle offenbart. Der hydraulische Kreislauf der Hämodilaysemaschine weist als eine Komponente einen Anschluss für Dialysatproben auf.

EP 1595560 A1 beschreibt eine Verfahren zur Überwachung der Zufuhr von Substitutionsflüssigkeit für einen Dialysators oder Filter, in einem Pre- oder Postdilutionsmodus.

FR 2 696 644 A beschreibt eine Vorrichtung zum Sammeln von Dialysatproben, wobei der Dialysatfluss in einem vorbestimmten Verhältnis in einen ersten und einen zweiten Teilfluss aufgeteilt wird, und wobei der kleinere der beiden Teilflüsse vollständig gesammelt und der größere der beiden Teilflüsse verworfen wird.

In der DE 42 03 069 A1 ist ein steriles Probenentnahmeset für die Dialyse beschrieben.

Der Erfindung liegt die Aufgabe zugrunde, ein gattungsgemäßes Hämodialysegerät oder Hämodiafiltrationsgerät derart weiterzubilden, dass eine einfache Probenentnahme der zubereiteten Dialysierflüssigkeit ohne aufwendige Hygienemaßnahmen ermöglicht wird. Der Erfindung liegt auch die Aufgabe zugrunde, ein entsprechendes Verfahren zur Probenentnahme bereitzustellen.

Nach der Lehre der Erfindung wird diese Aufgabe durch ein Hämodialysegerät mit den Merkmalen des Anspruches 1, durch ein Hämodiafiltrationsgerät mit den Merkmalen des Anspruchs 8, durch die Verfahren zur Probenentnahme mit den Merkmalen der Ansprüche 20 und 21 gelöst. Vorteilhafte Ausführungsformen der Erfindung sind Gegenstand der Unteransprüche.

Die Erfindung baut auf der Beobachtung auf, dass bei einem Hämodialyse- oder Hämodiafiltrationsgerät bereits vorhandene Anschlüsse zur Verbindung mit einem Hämodialysator oder zur Bereitstellung von Substitutionsflüssigkeit direkt auch für die Probenentnahme selbst genutzt werden können, da diese Anschlüsse vor der Aufrüstung mit den meist als Disposable ausgebildeten Komponenten des extrakorporalen Blutkreislaufs direkt zur Probenentnahme zugänglich sind. Diese Anschlüsse werden im Rahmen des Maschinenreinigungsprogramms regelmäßig desinfiziert und durch die Anschlüsse fließt während einer Dialysebehandlung genau die Flüssigkeit, deren Proben es zu analysieren gilt.

Durch Anschluss eines passenden sterilen Probenentnahmesets können einfach Proben entnommen werden, ohne dass besondere zusätzliche Hygienemaßnahmen erforderlich sind oder die Gefahr der Sekundärkontamination besteht. Auf der Geräteseite ist neben einer Einrichtung eines speziellen Probenentnahmesteuerprogramms in der sowieso vorhandenen Steuereinheit keinerlei Modifikation notwendig.

Weitere Einzelheiten und Vorteile der Erfindung werden anhand eines in den Zeichnungen schematisch dargestellten Ausführungsbeispiels näher beschrieben. Es zeigen:
Fig. 1 eine Ausführungsform eines erfindungsgemäßen Hämodiafiltrationsgerätes und
Fig. 2 eine Ausführungsform eines erfindungsgemäßen Probenentnahmesets.

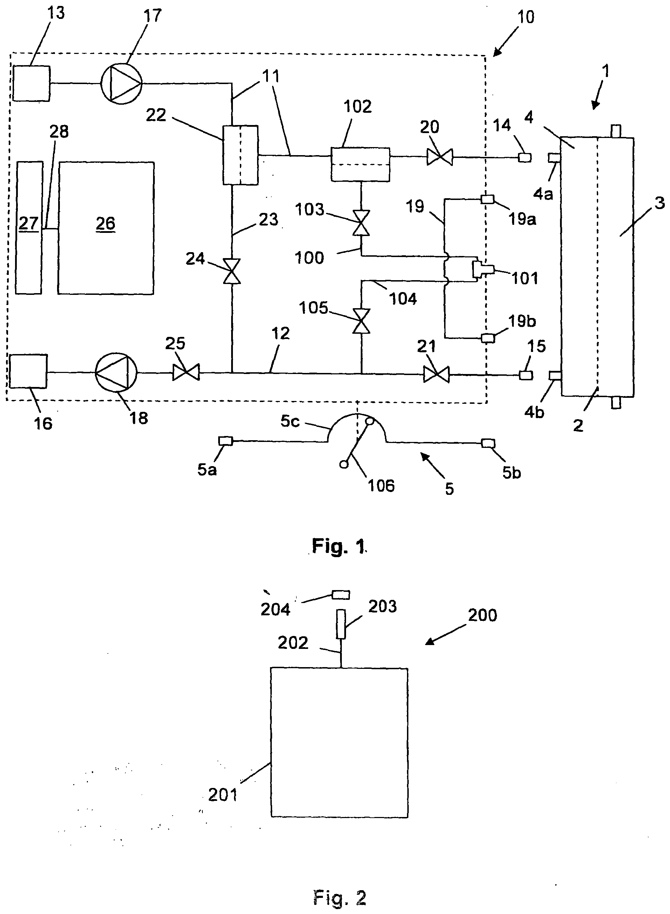

Mit Hilfe von Fig. 1 wird zunächst der prinzipielle Aufbau eines erfindungsgemäßen Hämodialysegerätes erläutert, wobei sich der Betrieb des in Fig. 1 gezeigten Hämodiafiltrationsgerätes 10 auf den Behandlungsmodus der Hämodialyse beschränkt. Danach schließt sich eine Beschreibung des Betriebs als erfindungsgemäßes Hämodiafiltrationsgerät an.

Zum Einsatz von Aktoren und Sensoren in einem Hämodialysegerät im Allgemeinen stehen dem Fachmann zahlreiche Möglichkeiten zur Verfügung, ohne dass hier im Detail darauf eingegangen werden muss. Die Darstellung in Fig. 1 ist auf einige wenige dieser Elemente beschränkt, die für die Erläuterung der Erfindung ausreichend sind.

Bei der Hämodialyse wird Blut in einem extrakorporalen Kreislauf einem Hämodialysator 1 zugeführt. Im Hämodialysator 1 trennt eine meist in Form vieler Hohlfasern ausgeführte semipermeable Membran 2 eine erste Kammer 3 (Blutkammer), die Teil des extrakorporalen Blutkreislaufs ist, von einer zweiten Kammer 4 (Dialysierflüssigkeitskammer), die Teil eines Dialysierflüssigkeitskreislaufes ist. Durch die semipermeable Membran 2 treten aus dem Blut zu entfernende Stoffe in die Dialysierflüssigkeit über, die durch diese abgeführt werden. Gleichzeitig kann über einen Druckgradienten eine überschüssige Flüssigkeitsmenge aus dem Blut ultrafiltriert und über die abfließende Dialysierflüssigkeit entfernt werden. Schließlich kann auch ein umgekehrter Diffusionsgradient zum Beispiel für Natriumionen verwendet werden, um diese Substanzen von der Dialysierflüssigkeit ins Blut zu überführen.

Die Blutkammer 3 ist über zwei Anschlussstutzen mit einer Blutzuführ- und einer Blutabführleitung (nicht gezeigt), die Dialysierflüssigkeitskammer 4 über zwei Anschlussstutzen 4a und 4b mit der Dialysierflüssigkeitszuführleitung 11 und einer Dialysierflüssigkeitsabführleitung 12 des Dialysierflüssigkeitskreislaufes des Hämodialyse- bzw. Hämodiafiltrationsgerätes 10 verbindbar. Die Dialysierflüssigkeitszuführleitung 11 führt von einer Dialysierflüssigkeitsquelle 13 zu einem ersten Anschlussmittel 14 zur Verbindung mit dem ersten Anschlussstutzen 4a der Dialysierflüssigkeitskammer 4, die Dialysierflüssigkeitsabführleitung 12 von einem zweiten Anschlussmittel 15 zur Verbindung mit dem zweiten Anschlussstutzen 4b der Dialysierflüssigkeitskammer 4 zu einem Abfluss 16. In der Dialysierflüssigkeitszuführleitung 11 ist eine erste Pumpeinrichtung 17 und in der Dialysierflüssigkeitsabführleitung 12 eine zweite Pumpeinrichtung 18 zum Umwälzen der Dialysierflüssigkeit sowie zur Steuerung des Flüssigkeitsentzugs während einer Dialysebehandlung vorgesehen. Die beiden Pumpeinrichtungen 17 und 18 sowie die Quelle 13 und der Abfluss 16 sind in Fig. 1 nur schematisch erfasst. Zu ihrer Umsetzung sind dem Fachmann zahlreiche Lösungen bekannt, wobei die Pumpeinrichtungen in weitem Sinn zu interpretieren sind. Sie können als getrennte, aktivfördernde Pumpen zum Einsatz kommen, es ist aber genauso möglich, ein sogenanntes Bilanzkammersystem mit separater Ultrafiltrationsleitung einzusetzen, wie es von der Patentanmelderin vertrieben wird und in der DE 28 38 414 C2 beschrieben ist, auf deren Offenbarungsgehalt explizit Bezug genommen wird.

Die Anschlussstutzen 4a und 4b sind bei handelsüblichen Hämodialysatoren nach DIN EN 1283 ausgebildet, wobei die ersten und zweiten Anschlussmittel 14 und 15 entsprechend komplementär und meist in Form so genannter Hansenkupplungen ausgeführt sind. Ist kein Hämodialysator 1 an das Hämodialysegerät 10 angeschlossen, werden die beiden Anschlussmittel 14 und 15, die über ein flexibles Schlauchstück an diesem Ende der Dialysierflüssigkeitszuführleitung 11 und der Dialysierflüssigkeitsabführleitung 12 mit dem Gehäuse des Hämodialysegerätes verbunden sind, über ein in dem Gerät integrierten Kurzschlussstück 19 mit Anschlüssen 19a und 19b kurzgeschlossen. Auf diese Weise kann der komplette Dialysierflüssigkeitskreislauf in einem Reinigungsverfahren zwischen einzelnen Blutbehandlungen mit einer Reinigungsflüssigkeit durchspült und gereinigt werden, ohne dass es zu Toträumen kommt. Lediglich der Hämodialysator wird üblicherweise nach einer Behandlung verworfen. Zwischen den Blutbehandlungen verbleibt der Dialysierflüssigkeitskreislauf grundsätzlich in diesem Kurzschlusszustand.

In der Dialysierflüssigkeitszuführleitung 11 ist ein als Schaltventil ausgeführtes erstes Verschlussmittel 20 und in der Dialysierflüssigkeitsabführleitung ein ähnliches zweites Verschlussmittel 21 vorgesehen. Mit den ersten und zweiten Verschlussmitteln 20 und 21 kann der Durchfluss in diesen Leitungen unterbrochen bzw. ermöglicht werden.

In der Dialysierflüssigkeitszuführleitung 11 kann ein erster Sterilfilter 22 vorgesehen sein, um frische Diaylsierflüssigkeit vor der Einleitung in den Hämodialysator zusätzlich zu filtern. Um die Standzeit des Filters zu erhöhen sowie die Gefahr einer Ruptur und damit der Einschwämmung von in dem Filter abgelagerten Keimen zu vermindern, kann der Filter wie in Fig. 1 gezeigt durch eine bei Hämodialysegeräten meist sowieso vorhandene Bypassleitung 23 spülbar sein, die die Dialysierflüssigkeitszuführleitung 11 mit der Dialysierflüssigkeitsabführleitung 12 verbindet. Zur Aktivierung des Bypasses ist in die Bypassleitung 23 ein drittes Verschlussmittel 24 geschaltet.

Die Bypassleitung 23 ermöglicht insbesondere in Fehlerfällen die Herbeiführung eines sicheren Zustandes während einer Hämodialysebehandlung. In diesem Fall werden die ersten und zweiten Verschlussmittel 20 und 21 geschlossen und das dritte Verschlussmittel 24 geöffnet. Das Hämodialysegerät 10 kann dabei die Aufbereitung und Verwerfung von Dialysat kontinuierlich fortsetzen, was eine Fortführung der Behandlung nach Behebung des Fehlers erleichtert.

Stromab der Mündung der Bypassleitung 23 in die Dialysierflüssigkeitsabführleitung 12 kann ein viertes Verschlussmittel 25 vorgesehen sein.

Das Hämodialysegerät 10 wird durch eine Steuereinheit 26 gesteuert und überwacht. Hierzu ist die Steuereinheit 26 mit den einzelnen Aktoren und Sensoren des Gerätes mit Signalleitungen verbunden, die jedoch der Übersichtlichkeit halber in Fig. 1 nicht gezeigt sind.

Wenn das Hämodialysegerät 10 auch zur Aufbereitung von Substitutionsflüssigkeit und damit als Hämodiafiltrationsgerät eingesetzt werden soll, zweigt von der Dialysierflüssigkeitszuführleitung 11 eine Substituatleitung 100 ab, die zu einem dritten Anschlussmittel 101 führt. In der in Fig. 1 dargestellten Ausführungsform wird die Abzweigung durch einen zweiten Sterilfilter 102 realisiert, dessen erste Kammer von der Dialysierflüssigkeit während der Behandlung durchströmt und damit ständig gespült wird. In die von der zweiten Kammer des zweiten Sterilfilters 102 abzweigende Substituatleitung 100 ist ein fünftes Verschlussmittel 103 geschaltet. Um die Substituatleitung 100 spülbar zu gestalten, kann eine von dem dritten Anschlussmittel 101 zur Dialysierflüssigkeitsabführleitung 12 führende Spülleitung 104 vorgesehen sein, in die ein sechstes Verschlussmittel 105 eingebaut ist. Die dritten Anschlussmittel 101 sind in diesem Fall als T-Stück ausgeführt, um eine Verbindung der Substituatleitung 100 mit dem extrakorporalen Kreislauf zu ermöglichen.

Für die Blutbehandlung würde ein als Wegwerfteil ausgeführtes Leitungsstück 5 mit einem komplementären Anschlussstück 5a an das dritte Anschlussmittel 101 angeschlossen werden. Das andere Leitungsende 5b wird, wenn es aufgrund der Gestaltung des extrakorporalen Kreislaufs nicht schon integral mit diesem verbunden ist, mit dem extrakorporalen Kreislauf an geeigneter Stelle verbunden. In dem Leitungsstück 5 kann ein Pumpsegment 5c zum Einlegen in eine als Rollenpumpe ausgeführte Pumpe 106 des Hämodiafiltrationsgerätes 10 vorgesehen sein.

Die Steuereinheit 26 ist mit einer Eingabe- und Ausgabeeinheit 27 über eine Datenleitung 28 verbunden. Die Eingabe- und Ausgabeeinheit 27 umfasst Mittel zum Aktivieren eines speziellen Probenentnahmesteuerprogramms in der Steuereinheit 26.

Zur Durchführung des erfindungsgemäßen Verfahrens zur Probenentnahme wird zunächst lediglich ein steriles Probenentnahmeset an das erste, zweite oder dritte Anschlussmittel 14, 15 oder 101 angeschlossen. Ein Ausführungsbeispiel eines solches Sets ist in Fig. 2 zu sehen.

Das sterile Probenentnahmeset 200 besteht aus einem vorzugsweise als Beutel ausgeführten Behältnismittel 201, das über einen Leitungsabschnitt 202 zu einem Behältnisanschlussmittel 203 führt. Weiterhin kann in den Leitungsabschnitt 202 oder an geeigneter Stelle ein als Schlauchklemme ausgebildetes Behältnisverschlussmittel vorgesehen sein, damit das Probenentnahmeset nach der Probenentnahme verschlossen werden kann. Geeignet als Behältnisverschlussmittel ist auch eine sterile Verschlusskappe 204, die als Teil des sterilen Probenentnahmesets 200 ausgeliefert werden kann. Durch Verschluss des Behältnisanschlussmittels 203 mit der Verschlusskappe 204 nach der Probenentnahme kann sichergestellt werden, dass die Probenflüssigkeit auf dem Weg zum Analyselabor nicht kontaminiert wird.

Falls das Probenentnahmeset 200 an die ersten oder zweiten Anschlussmittel 14 oder 15 angeschlossen werden soll, ist das Behältnisanschlussmittel 203 als Anschlussport nach DIN EN 1283 ähnlich der Stutzen 4a, 4b des Hämodialysators 1 ausgestaltet. Im Falle eines Anschlusses an das dritte Anschlussmittel 101 ist es entsprechend komplementär zu diesem Anschlussmittel ausgebildet, wobei je nach Hersteller verschiedene Konnektortypen eingesetzt werden. Besonders vielseitig wird das erfindungsgemäße Probenentnahmeset, wenn das Behältnisanschlussmittel 203 mit beiden Anschlussformen z.B. in Form eines Y-Stückes ausgestattet ist. Es kann auch vorgesehen sein, dass über ein entfernbares Adapterstück die Kompatibiltät mit beiden Anschlusstypen hergestellt werden kann.

Das Probenentnahmeset 200 liegt zur Vermeidung von Sekundärkontaminationen in steriler Form vor. Hierzu kann es ausreichend sein, dass die Innenflächen steril sind, wenn ausgeschlossen werden kann, dass die von außen zugänglichen Flächen zu Kontaminationen führen. Zweckmäßigerweise wird man das Probenentnahmeset aber in einer Umverpackung vollständig sterilisieren und dann in der Umverpackung in Umlauf bringen.

Neben der Ausführungsform als Beutel sind auch andere Gestaltungsmöglichkeiten möglich. So kann auch eine sterile Spritze mit entsprechenden Anschlussmitteln Verwendung finden.

Bevor das Probenentnahmeset an dem Hämodiafiltrationsgerät 10 angeschlossen wird, wählt der Bediener über entsprechende Eingabemittel der Eingabe- und Ausgabeeinheit 27 ein spezielles Probenentnahmesteuerprogramm an. Im Rahmen dieses automatisch in der Steuereinheit 26 ablaufenden Programms können daraufhin wie nachfolgend beschrieben alternative Verfahrensschritte ablaufen.

Im Falle eines Anschlusses an das erste oder zweite Anschlussmittel schließt das Programm zunächst die ersten und zweiten Verschlussmittel 20 und 21 oder hält sie geschlossen, falls sie bereits geschlossen sind. Der Bediener wird dann zum Anschluss des Probenentnahmesets über die Eingabe- und Ausgabeeinheit 27 aufgefordert. Nach dem Anschließen des Probenentnahmesets 200 an das erste oder zweite Anschlussmittel bestätigt der Bediener den Anschluss an der Eingabe- und Ausgabeeinheit 27, falls erforderlich erfolgt auch eine Auswahl des verwendeten Anschlusses. Das Steuerprogramm in der Steuereinheit 26 öffnet daraufhin das vorbestimmte oder ausgewählte erste oder zweite Verschlussmittel 20 oder 21, an dem das Probenentnahmeset angeschlossen ist, und betätigt die Pumpeinrichtung 17, damit eine vorgegebene Menge von Dialysierflüssigkeit in das Behältnis 201 fließen kann. Anschließend wird das geöffnete erste oder zweite Verschlussmittel 20 oder 21 wieder geschlossen. Der Abschluss des Probenentnahmesteuerprogramms wird auf der Eingabe- und Ausgabeeinheit 27 angezeigt. Das Probenentnahmeset kann nun entnommen werden.

In einer Weiterbildung der Erfindung kann vorgesehen sein, dass das Hämodiafiltrationsgerät einen Beschriftungsausdruck automatisch zur Verfügung stellt, der auf dem Probenentnahmeset aufgebracht werden kann und die notwendigen Daten wie Gerätenummer und Zeitpunkt der Probennahme bereits enthält.

Das Probenentnahmesteuerprogramm kann je nach Aufbau des Hämodiafiltrationsgerätes verschiedene Ventilschaltungen für die Probenentnahme einsetzen. Bei dem in der Fig. 1 gezeigten Gerät können bei Anschluss am ersten Anschlussmittel 14 alle Verschlussmittel geschlossen sein, und für die Probennahme braucht nur das erste Verschlussmittel 20 geöffnet zu werden. Bei Anschluss an die zweiten Anschlussmittel 15 dagegen ist neben der Öffnung des zweiten Verschlussmittels 21 auch eine Öffnung eines Verschlussmittels in einer die Dialysierflüssigkeitszuführleitung 11 mit der Dialysierflüssigkeitsabführleitung 12 verbindenden Leitung notwendig. In der Ausführungsform von Fig. 1 kann hierfür die Bypassleitung 23 bei Öffnung der dritten Verschlussmittel 24 oder die Substituatleitung 100 und die Spülleitung 104 unter Öffnung der fünften und sechsten Verschlussmittel 103 und 105 verwendet werden. Das vierte Verschlussmittel 25 ist zweckmäßigerweise geschlossen.

Bei Anschluss an das dritte Anschlussmittel 101 können die ersten und zweiten Verschlussmittel 20 und 21 im Falle eines Kurzschlusses der ersten und zweiten Anschlussmittel 14 und 15 auch geöffnet sein. Hierzu können Sensoren an den Anschlüssen 19a und 19b des Kurzschlussstückes 19 vorgesehen sein, die generell das Vorhandensein einer Konnektion erfassen können. Zur Probenentnahme wird das fünfte Verschlussmittel 103 oder das dritte und das sechste Verschlussmittel 24 und 105 geöffnet.

Unabhängig von der realisierten Alternative können entsprechende Ein- und Ausgaberoutinen der Eingabe- und Ausgabeeinheit 27 in der bereits beschriebenen Weise die einfache Aktivierung des Probenentnahmeprogramms sowie die Bestätigung der einzelnen Verfahrensschritte und ggf. die Auswahl eines bestimmten Flüssigkeitsverlaufs und/oder Anschlussmittels durch den Anwender ermöglichen, wodurch sich die Durchführung des erfindungsgemäßen Verfahrens mit Hilfe des erfindungsgemäßen Hämodialyse- oder Hämodiafiltrationsgerätes als besonders anwenderfreundlich gestaltet.

Durch die Erfindung ist eine einfache Probenentnahme von durch Hämodialyse- oder Hämodiafiltrationsgeräte hergestellter und gebrauchsfertiger Dialysierflüssigkeit möglich, ohne dass es des Einbaus zusätzlicher Komponenten sowie der Einhaltung aufwendiger Hygienemaßnahmen bedarf. Gleichzeitig werden Sekundärkontaminationen effektiv vermieden. Das ebenfalls erfindungsgemäße Probenentnahmeset kann als Kunststoffartikel kostengünstig in großer Stückzahl steril hergestellt werden. Durch die Anschlusskompatibilität des Probenentnahmesets mit den bereits vorhandenen Anschlüssen für die gebrauchsfertige Dialysierflüssigkeit wird ein lange existierendes Bedürfnis nach einer einfachen und hygienisch nicht anfälligen Möglichkeit der routinemäßigen Probenentnahme für mikrobiologische Untersuchungen befriedigt.

## Patentansprüche

1. Hämodialysegerät (10) mit einem Dialysierflüssigkeitskreislauf, der eine Quelle (13) und einen Abfluss (16) für Dialysierflüssigkeit, eine von der Quelle (13) für Dialysierflüssigkeit zu einem ersten Anschlussmittel (14) führende Dialysierflüssigkeitszuführleitung (11), eine von einem zweiten Anschlussmittel (15) zu dem Abfluss (16) für Dialysierflüssigkeit führende Dialysierflüssigkeitsabführleitung (12), eine Pumpeinrichtung (17, 18) zum Umwälzen von Dialysierflüssigkeit in dem Dialysierflüssigkeitskreislauf sowie erste Verschlussmittel (20) in der Dialysierflüssigkeitszuführleitung (11) und zweite Verschlussmittel (21) in der Dialysierflüssigkeitsabführleitung (12), und ferner eine Steuereinheit (26) zum Ansteuern der Pumpeinrichtung (17, 18) und der ersten und zweiten Verschlussmittel (20, 21) aufweist, und mit einem an die ersten oder die zweiten Anschlussmittel (14, 15) angeschlossenen Probenentnahmeset (200) mit einem komplementär zu dem ersten oder dem zweiten Anschlussmittel (14, 15) ausgeführten Behältnisanschlussmittel (203), wobei die ersten und zweiten Anschlussmittel (14, 15) so ausgebildet sind, dass sie bei der Durchführung einer Hämodialysebehandlung mit ersten und zweiten Anschlussstutzen einer Dialysierflüssigkeitskammer (4) eines durch eine semipermeable Membran (2) in eine Dialysierflüssigkeitskammer (4) und eine Blutkammer (3) geteilten Hämodialysators (1) verbunden werden können und komplementär zu den ersten und zweiten Anschlussstutzen ausgeführt sind, damit Dialysierflüssigkeit von der Quelle (13) über die Dialysierflüssigkeitszuführleitung (11) zur Dialysierflüssigkeitskammer (4) und von der Dialysierflüssigkeitskammer (4) über die Dialysierflüssigkeitsabführleitung (12) zum Abfluss (16) fließen kann, wobei die Steuereinheit (26) so ausgebildet ist, dass sie im Rahmen eines automatisch ablaufenden Probenentnahmesteuerprogramms die ersten und zweiten Verschlussmittel (20, 21) sowie die Pumpeinrichtung (17, 18) so ansteuert, dass eine vorgegebene Menge Dialysierflüssigkeit in das an den ersten oder zweiten Anschlussmitteln (14, 15) angeschlossene Probenentnahmeset (200) gefördert wird.

2. Hämodialysegerät nach Anspruch 1, wobei das Probenentnahmeset steril ist, aus einem Behältnis (201) und einem von dem Behältnis (201) zu dem Behältnisanschlussmittel (203) führenden Leitungsabschnitt (202).

3. Hämodialysegerät nach Anspruch 1 oder 2, wobei die ersten und zweiten Anschlussmittel (14, 15) Kupplungen passend für Dialysierflüssigkeitsanschlussstutzen (4a, 4b) nach DIN EN 1283 sind.

4. Hämodialysegerät nach einem der vorangehenden Ansprüche, wobei das Probenentnahmesteuerprogramm zur Förderung der vorgegebenen Menge Dialysierflüssigkeit zunächst beide Verschlussmittel (20, 21) schließt oder geschlossen hält und anschließend das erste Verschlussmittel (20) öffnet und die Pumpeinrichtung (17) zur Förderung der vorgegebenen Menge Dialysierflüssigkeit in das an den ersten Anschlussmitteln (14) angeschlossene Probenentnahmeset betreibt und danach das erste Verschlussmittel (20) wieder schließt.

5. Hämodialysegerät nach einem der vorhergehenden Ansprüche, das ferner eine stromauf von dem ersten Verschlussmittel (20) von der Dialysierflüssigkeitzuführleitung (11) abzweigende Bypassleitung (23) aufweist, die mit einem dritten Verschlussmittel (24) versehen ist und in die Dialysierflüssigkeitsabführleitung (12) stromab des zweiten Verschlussmittels (21) mündet, wobei in der Dialysierflüssigkeitsabführleitung (12) stromab des Mündungspunktes ein viertes Verschlussmittel (25) vorgesehen ist

6. Hämodialysegerät nach Anspruch 5, wobei das Probenentnahmesteuerprogramm zur Förderung der vorgegebenen Menge Dialysierflüssigkeit zunächst das erste (20), zweite (21) und vierte (25) Verschlussmittel schließt oder geschlossen hält und anschließend das dritte Verschlussmittel (24) öffnet oder geöffnet hält und das zweite Verschlussmittel (21) öffnet sowie danach die Pumpeinrichtung (17) zur Förderung der vorgegebenen Menge Dialysierflüssigkeit in das an den zweiten Anschlussmitteln (15) angeschlossene Probenentnahmeset betreibt und danach das zweite Verschlussmittel (21) wieder schließt.

7. Hämodialysegerät nach einem der vorhergehenden Ansprüche zur Verwendung als Hämodiafiltrationsgerät, wobei der Dialysierflüssigkeitskreislauf eine von der Dialysierflüssigkeitszuführleitung (11) zu einem dritten Anschlussmittel (101) abzweigende Substituatleitung (100) sowie fünfte Verschlussmittel (103) in der Substituatleitung (100) aufweist und wobei die dritten Anschlussmittel (101) so ausgebildet sind, dass bei der Durchführung einer Hämodiafiltrationsbehandlung Dialysierflüssigkeit über eine an dem dritten Anschlussmittel (101) angeschlossene Leitung (5) dem Blut eines Patienten als Substituat zugeführt wird, und wobei die Steuereinheit (26) so ausgebildet ist, dass sie im Rahmen des automatisch ablaufenden Probenentnahmesteuerprogramms auch die fünften (103) Verschlussmittel so ansteuert, dass die vorgegebene Menge Dialysierflüssigkeit in das an den ersten (14), oder zweiten (15) Anschlussmitteln angeschlossene Probenentnahmeset (200) gefördert wird.

8. Hämodiafiltrationsgerät mit einem Dialysierflüssigkeitskreislauf, der eine Quelle (13) und einen Abfluss (16) für Dialysierflüssigkeit, eine von der Quelle (13) für Dialysierflüssigkeit zu einem ersten Anschlussmittel (14) führende Dialysierflüssigkeitszuführleitung (11), eine von einem zweiten Anschlussmittel (15) zu dem Abfluss (16) für Dialysierflüssigkeit führende Dialysierflüssigkeitsabführleitung (12), eine von der Dialysierflüssigkeitszuführleitung (11) zu einem dritten Anschlussmittel (101) abzweigende Substituatleitung (100), eine Pumpeinrichtung (17, 18) zum Umwälzen von Dialysierflüssigkeit in dem Dialysierflüssigkeitskreislauf sowie erste Verschlussmittel (20) in der Dialysierflüssigkeitszuführleitung (11) und zweite Verschlussmittel (21) in der Dialysierflüssigkeitsabführleitung (12) und fünfte Verschlussmittel (103) in der Substituatleitung (100), und ferner eine Steuereinheit (26) zum Ansteuern der Pumpeinrichtung (17, 18) und der ersten (20), zweiten (21) und fünften (103) Verschlussmittel aufweist, und mit einem an das dritte Anschlussmittel (101) angeschlossenen Probenentnahmeset (200), wobei die ersten (14) und zweiten (15) Anschlussmittel so ausgebildet sind, dass sie bei der Durchführung einer Hämodiafiltrationsbehandlung mit der Dialysierflüssigkeitskammer (4) eines durch eine semipermeable Membran (2) in eine Dialysierflüssigkeitskammer (4) und eine Blutkammer (3) geteilten Hämodialysators (1) verbunden werden können, damit Dialysierflüssigkeit von der Quelle (13) über die Dialysierflüssigkeitszuführleitung (11) zur Dialysierflüssigkeitskammer (4) und von der Dialysierflüssigkeitkammer (4) über die Dialysierflüssigkeitsabführleitung (12) zum Abfluss (16) fließt, und die dritten Anschlussmittel (101) so ausgebildet sind, dass bei der Durchführung einer Hämodiafiltrationsbehandlung Dialysierflüssigkeit über eine an dem dritten Anschlussmittel (101) angeschlossene Leitung (5) dem Blut eines Patienten als Substituat zugeführt werden kann, wobei die Steuereinheit (26) so ausgebildet ist, dass sie im Rahmen eines automatisch ablaufenden Probenentnahmesteuerprogramms die ersten (20), zweiten (21) und fünften (103) Verschlussmittel sowie die Pumpeinrichtung (17, 18) so ansteuert, dass eine vorgegebene Menge Dialysierflüssigkeit in das an den dritten (101) Anschlussmitteln angeschlossene Probenentnahmeset (200) gefördert wird.

9. Hämodialfiltrationsgerät nach Anspruch 8, wobei das Probenentnahmeset steril ist, aus einem Behältnis (201) und einem von dem Behältnis (201) zu dem Behältnisanschlussmittel (203) führenden Leitungsabschnitt (202) besteht und über ein Behältnisanschlussmittel (203) an das dritte Anschlussmittel (101) angeschlossen ist.

10. Hämodiafiltrationsgerät nach einem der Ansrüche 7 bis 9, wobei die ersten (14) und zweiten (15) Anschlussmittel Kupplungen passend für Dialysierflüssigkeitsanschlussstutzen (4a, 4b) nach DIN EN 1283 sind.

11. Hämodiafiltrationsgerät nach einem der Ansprüche 7 bis 10, wobei das Probenentnahmesteuerprogramm zur Förderung der vorgegebenen Menge Dialysierflüssigkeit zunächst das erste (20), zweite (21) und fünfte (103) Verschlussmittel schließt oder geschlossen hält und anschließend das fünfte (103) Verschlussmittel öffnet sowie danach die Pumpeinrichtung (17) zur Förderung der vorgegebenen Menge Dialysierflüssigkeit in das an den dritten Anschlussmitteln (101) angeschlossene Probenentnahmeset betreibt und anschließend das fünfte Verschlussmittel (103) wieder schließt.

12. Hämodiafiltrationsgerät nach einem der Ansprüche 7 bis 11, wobei das Probenentnahmesteuerprogramm zur Förderung der vorgegebenen Menge Dialysierflüssigkeit zunächst das erste (20), zweite (21) und fünfte (103) Verschlussmittel schließt oder geschlossen hält und anschließend das erste Verschlussmittel (20) öffnet und die Pumpeinrichtung (17) zur Förderung der vorgegebenen Menge Dialysierflüssigkeit in das an den ersten Anschlussmitteln (14) angeschlossene Probenentnahmeset (200) betreibt und danach das erste Verschlussmittel (20) wieder schließt.

13. Hämodiafiltrationsgerät nach einem der Ansprüche 7 bis 12, wobei es ferner eine stromauf von dem ersten Verschlussmittel (20) von der Dialysierflüssigkeitszuführleitung (11) abzweigende Bypassleitung (23) aufweist, die mit einem dritten Verschlussmittel (24) versehen ist und in die Dialysierflüssigkeitsabführleitung (12) stromab des zweiten Verschlussmittels (21) mündet, wobei in der Dialysierflüssigkeitsabführleitung (12) stromab des Mündungspunktes ein viertes Verschlussmittel (25) vorgesehen ist.

14. Hämodiafiltrationsgerät nach Anspruch 13, wobei das Probenentnahmesteuerprogramm zur Förderung der vorgegebenen Menge Dialysierflüssigkeit zunächst das erste (20), zweite (21), vierte (25) und fünfte (103) Verschlussmittel schließt oder geschlossen hält und anschließend das dritte Verschlussmittel (24) öffnet oder geöffnet hält und das zweite Verschlussmittel (21) öffnet sowie danach die Pumpeinrichtung (17) zur Förderung der vorgegebenen Menge Dialysierflüssigkeit in das an den zweiten Anschlussmitteln angeschlossene Probenentnahmeset (200) betreibt und danach das zweite Verschlussmittel (21) wieder schließt.

15. Hämodiafiltrationsgerät nach einem der Ansprüche 6 bis 14, wobei das dritte Anschlussmittel (101) mit einem Deckel verschlossen werden und die mit Flüssigkeit in Kontakt kommende Innenfläche des dritten Anschlussmittels (101) über die abzweigende Substituatleitung (100) und einer von dem dritten Anschlussmittel (101) wegführenden Spülleitung (104) im Durchfluss gereinigt werden kann.

16. Hämodiafiltrationsgerät nach Anspruch 15, wobei die Spülleitung (104) stromabwärts des zweiten Verschlussmittels (21) in die Dialysierflüssigkeitsabführleitung (12) mündet und mit einem sechsten Verschlussmittel (105) versehen ist.

17. Hämodialysegerät nach einem der Ansprüche 1 - 7 oder Hämodiafiltrationsgerät nach einem der Ansprüche 8 - 16 wobei das Behältnisanschlussmittel (203) ein Port entsprechend einem Dialysierflüssigkeitsanschlussstutzen nach DIN EN 1283 ist.

18. Hämodialysegerät nach einem der Ansprüche 1 - 7 oder 17 oder Hämodiafiltrationsgerät nach einem der Ansprüche 8 - 16 oder 17, wobei das Behältnis (201) ein flexibler Beutel ist.

19. Hämodialysegerät nach einem der Ansprüche 1 - 7, 17 oder 18 oder Hämodiafiltrationsgerät nach einem der Ansprüche 8 - 16, 17 oder 18, wobei das sterile Probenentnahmeset sterile Behältnisverschlussmittel (204) zum Verschließen des Probenentnahmesets nach der Probenentnahme umfasst.

20. Verfahren zur Probennahme von Dialysierflüssigkeit eines Hämodialysegerätes, das einen Dialysierflüssigkeitskreislauf, der eine Quelle (13) und einen Abfluss (16) für Dialysierflüssigkeit, eine von der Quelle (13) für Dialysierflüssigkeit zu einem ersten Anschlussmittel (14) führende Dialysierflüssigkeitszuführleitung (11), eine von einem zweiten Anschlussmittel (15) zu dem Abfluss (16) für Dialysierflüssigkeit führende Dialysierflüssigkeitsabführleitung (12), eine Pumpeinrichtung (17, 18) zum Umwälzen von Dialysierflüssigkeit in dem Dialysierflüssigkeitskreislauf sowie erste Verschlussmittel (20) in der Dialysierflüssigkeitszuführleitung (11) und zweite Verschlussmittel (21) in der Dialysierflüssigkeitsabführleitung (12) aufweist, wobei die ersten (14) und zweiten (15) Anschlussmittel so ausgebildet sind, dass sie bei der Durchführung einer Hämodialysebehandlung mit der Dialysierflüssigkeitskammer (4) eines durch eine semipermeable Membran (2) in eine Dialysierflüssigkeitskammer (4) und eine Blutkammer (3) geteilten Hämodialysators (1) verbunden werden, damit Dialysierflüssigkeit von der Quelle (13) über die Dialysierflüssigkeitszuführleitung (11) zur Dialysierflüssigkeitskammer (4) und von der Dialysierflüssigkeitkammer (4) über die Dialysierflüssigkeitsabführleitung (12) zum Abfluss (16) fließt,
mit folgenden Verfahrensschritten:
Schließen oder geschlossen Halten der beiden Verschlussmittel (20, 21),
Anschließen eines sterilen Probenentnahmesets (200) an das erste (14) oder zweite (15) Anschlussmittel,
Öffnen des ersten (20) oder zweiten (21) Verschlussmittels, an dem das Probenentnahmeset (200) angeschlossen ist,
Betätigen der Pumpeinrichtung (17, 18), damit eine vorgegebene Menge von Dialysierflüssigkeit in das Probenentnahmeset (200) fließt,
Schließen des ersten (20) oder zweiten (21) Verschlussmittels.

21. Verfahren zur Probennahme von Dialysierflüssigkeit eines Hämodiafiltrationsgeräts, das einen Dialysierflüssigkeitskreislauf, der eine Quelle (13) und einen Abfluss (16) für Dialysierflüssigkeit, eine von der Quelle (13) für Dialysierflüssigkeit zu einem ersten Anschlussmittel (14) führende Dialysierflüssigkeitszuführleitung (11), eine von einem zweiten Anschlussmittel (15) zu dem Abfluss (16) für Dialysierflüssigkeit führende Dialysierflüssigkeitsabführleitung (12), eine von der Dialysierflüssigkeitszuführleitung (11) zu einem dritten Anschlussmittel (101) abzweigende Substituatleitung (100), eine Pumpeinrichtung (17, 18) zum Umwälzen von Dialysierflüssigkeit in dem Dialysierflüssigkeitskreislauf sowie erste Verschlussmittel (20) in der Dialysierflüssigkeitszuführleitung (11) und zweite Verschlussmittel (21) in der Dialysierflüssigkeitsabführleitung (12) und fünfte Verschlussmittel (103) in der Substituatleitung (100) aufweist, wobei die ersten (14) und zweiten (15) Anschlussmittel so ausgebildet sind, dass sie bei der Durchführung einer Hämodiafiltrationsbehandlung mit der Dialysierflüssigkeitskammer (4) eines durch eine semipermeable Membran (2) in eine Dialysierflüssigkeitskammer (4) und eine Blutkammer (3) geteilten Hämodialysators (1) verbunden werden, damit Dialysierflüssigkeit von der Quelle (13) über die Dialysierflüssigkeitszuführleitung (11) zur Dialysierflüssigkeitskammer (4) und von der Dialysierflüssigkeitkammer (4) über die Dialysierflüssigkeitsabführleitung (12) zum Abfluss (16) fließt, und die dritten Anschlussmittel (101) so ausgebildet sind, dass bei der Durchführung einer Hämodiafiltrationsbehandlung Dialysierflüssigkeit über eine an dem dritten Anschlussmittel (101) angeschlossene Leitung (5) dem Blut eines Patienten als Substituat zugeführt wird,
mit folgenden Verfahrensschritten:
Schließen oder geschlossen Halten der ersten (20) und zweiten (21) Verschlussmittel oder Kurzschließen der ersten (14) und zweiten (15) Anschlussmittel,
Schließen oder geschlossen Halten der fünften Verschlussmittel (103),
Anschließen eines sterilen Probenentnahmesets (200) an das dritte Anschlussmittel (101),
Öffnen des fünften Verschlussmittels (103),
Betätigen der Pumpeinrichtung (17, 18), damit eine vorgegebene Menge von Dialysierflüssigkeit in das Probenentnahmeset (200) fließt,
Schließen des fünften Verschlussmittels (103).

## Claims

1. A hemodialysis machine (10) having a dialysis fluid circulation which includes a source (13) and an outlet (16) for the dialysis fluid, a dialysis fluid supply line (11) leading from the source (13) for dialysis fluid to a first connecting means (14), a dialysis fluid drain line (12) leading from a second connecting means (15) to the outlet (16) for dialysis fluid, a pumping mechanism (17, 18) for circulating dialysis fluid in the dialysis fluid circulation and first closure means (20) in the dialysis fluid supply line (11) and second closure means (21) in the dialysis fluid outlet line (12) and also a control unit (26) for controlling the pumping mechanism (17, 18) and the first and second closure means (20, 21), and having a sampling set (200) which is connected to the first or second connecting means (14, 15) with a container connecting means (203) designed to be complementary to the first or second connecting means (14, 15), wherein the first and second connecting means (14, 15) are designed so that when a hemodialysis treatment is being carried out, these connecting means can be connected to the first and second connections of a dialysis fluid chamber (4) of a hemodialyser (1) which is divided by a semipermeable membrane (2) into a dialysis fluid chamber (4) and a blood chamber (3), and
are designed to be complementary to the first and second connections so that dialysis fluid can flow from the source (13) through the dialysis fluid supply line (11) to the dialysis fluid chamber (4) and from the dialysis fluid chamber (4) through the dialysis fluid outlet line (12) to the outlet (16),
wherein the control unit (26) is designed so that it controls the first and second closure means (20, 21) and the pumping mechanism (17, 18) as part of a sampling control program that runs automatically so that a predetermined amount of dialysis fluid is conveyed into the sampling set (200) connected to the first or second connection means (14, 15).

2. The hemodialysis machine according to claim 1, wherein the sampling set is sterile, consisting of a container (201) and a line section (202) leading from the container (201) to the container-connecting means (203).

3. The hemodialysis machine according to claim 1 or 2, wherein the first and second connecting means (14, 15) are couplings that fit dialysis fluid connections (4a, 4b) according to DIN EN 1283.

4. The hemodialysis machine according to any one of the preceding claims, wherein the sampling control program for conveying the predefined quantity of dialysis fluid first closes or keeps closed both of the closure means (20, 21) and then opens the first closure means (20) and operates the pump system (17) for conveying the predefined amount of dialysis fluid into the sampling set connected to the first connecting means (14) and then closes the first closure means (20) again.

5. The hemodialysis machine according to any one of the preceding claims, also having a bypass line (23), which branches off from the dialysis fluid supply line (11) upstream from the first closure means (20) and is provided with a third closure means (24) and opens into the dialysis fluid outlet line (12) downstream from the second closure means (21), wherein a fourth closure means (25) is provided in the dialysis fluid outlet line (12) downstream from the mouth opening point.

6. The hemodialysis machine according to claim 5, wherein the sampling control program for conveying the predefined amount of dialysis fluid first closes or keeps closed the first closure means (20), the second closure means (21) and the fourth closure means (25) and then opens or keeps open the third closure means (24) and opens the second closure means (21) as well as thereafter operating the pumping mechanism (17) for conveying the predefined amount of dialysis fluid into the sampling set connected to the second connecting means (15) and then closes the second closure means (21) again.

7. The hemodialysis machine according to any one of the preceding claims for use as a hemodiafiltration machine, wherein the dialysis fluid circulation has a substituate line (100) leading from the dialysis fluid supply line (11) to a third connecting means (101) as well as having fifth closure means (103) in the substituate line (100), and wherein the third closure means (101) are designed so that, when carrying out a hemodiafiltration treatment, the dialysis fluid is supplied as a substituate to a patient's blood through a line (5) connected to the third connecting means (101), and wherein the control unit (26) is designed so that it also controls the fifth closure means (103) as part of the sampling control program, which takes place automatically, so that the predefined amount of dialysis fluid is conveyed into the sampling set (200) connected to the first connecting means (14) or the second connecting means (15).

8. A hemodiafiltration machine having a dialysis fluid circulation which comprises a source (13) and an outlet (16) for dialysis fluid, a dialysis fluid supply line (11) leading from the source (13) for dialysis fluid to a first connecting means (14), a dialysis fluid outlet line (12) leading from the second connecting means (15) to the outlet (16) for dialysis fluid, a substituate line (100) branching off from the dialysis fluid supply line (11) to a third connecting means (101), a pumping mechanism (17, 18) for circulating dialysis fluid in the dialysis fluid circulation as well as first closure means (20) in the dialysis fluid supply line (11) and second closure means (21) in the dialysis fluid outlet line (12) and fifth closure means (103) in the substituate line (100) and also a control until (26) for controlling the pumping mechanism (17, 18) and the first closure means (20), the second closure means (21) and the fifth closure means (103), and having a sampling set (200) connected to the third connecting means (101), wherein the first connecting means (14) and the second connecting means (15) are designed, so that, while carrying out a hemodiafiltration treatment with the dialysis fluid chamber (4) of a hemodialyser (1) divided by a semipermeable membrane (2) into a dialysis fluid chamber (4) and a blood chamber (3), it can be connected to the dialysis fluid chamber (4), so that
dialysis fluid flows from the source (13) through the dialysis fluid supply line (11) to the dialysis fluid chamber (4) and from the dialysis fluid chamber (4) through the dialysis fluid outlet line (12) to the outlet (16), and the third connecting means (101) are designed, so that in carrying out a hemodiafiltration treatment, dialysis fluid can be added as a substituate to a patient's blood through a line (5) connected to the third connecting means (101),
wherein the control unit (26) is designed so that, within the context of a sampling control program running automatically, the control unit controls the first closure means (20), the second closure means (21) and the fifth closure means (103) as well as the pump system (17, 18), so that a predefined amount of dialysis fluid is conveyed into the sampling set (200) connected to the third connecting means (101).

9. The hemodiafiltration machine according to claim 8, wherein the sampling set is sterile, consisting of a container (201) and a line section (202) leading from the container (201) to the container-connecting means (203) and connected to the third connecting means (101) via a container-connecting means (203).

10. The hemodiafiltration machine according to any one of claims 7 to 9, wherein the first connecting means (14) and the second connecting means (15) are couplings which fit dialysis fluid connections (4a, 4b) according to DIN EN 1283.

11. The hemodiafiltration machine according to any one of claims 7 to 10, wherein the sampling control program for conveying the predefined amount of dialysis fluid first closes or keeps closed the first closure means (20), the second closure means (21) and the fifth closure means (103) and then opens the fifth closure means (103) as well as thereafter operating the pump system (17) for conveying the predefined amount of dialysis fluid into the sampling set connected to the third connecting means (101) and then closes the fifth closure means (103) again.

12. The hemodiafiltration machine according to any one of claims 7 to 11, wherein the sampling control program for conveying the predefined amount of dialysis fluid first closes the first closure means (20), the second closure means (21) and the fifth closure means (103) or keeps them closed and then opens the first closure means (20) and operates the pump system (17) for conveying the predefined amount of dialysis fluid into the sampling set (200) connected to the first connecting means (14) and then closes the first closure means (20) again.

13. The hemodiafiltration machine according to any one of claims 7 to 12, wherein it also has a bypass line (23), which branches off from the dialysis fluid supply line (11) upstream from the first closure means (20) and is provided with a third closure means (24), opening into the dialysis fluid outlet line (12) downstream from the second closure means (21), wherein a fourth closure means (25) is provided in the dialysis fluid outlet line (12) downstream from the opening point.

14. The hemodiafiltration machine according to claim 13, wherein the sampling control program for conveying the predefined amount of dialysis fluid first closes or keeps closed the first closure means (20), the second closure means (21), the fourth closure means (25) and the fifth closure means (103) and then opens the third closure means (24) or keeps it open and opens the second closure means (21) as well as thereafter operating the pump system (17) for conveying the predefined amount of dialysis fluid into the sampling set (200) connected to the second connecting means and then closes the second closure means (21) again.

15. The hemodiafiltration machine according to any one of claims 6 to 14, wherein the third connecting means (101) are closed with a cover, and the inside surface of the first connecting means (101) coming in contact with fluid can be cleaned in continuous flow via the substituate line (100) branching off and via a rinsing line (104) leading away from the third connecting means (101) in continuous flow.

16. The hemodiafiltration machine according to claim 15, wherein the rinse line (104) of the second closure means (21) opens into the dialysis fluid outlet line (12) and is provided with a sixth closure means (105).

17. The hemodialysis machine according to any one of claims 1-7 or the hemodiafiltration machine according to any one of claims 8-16, wherein the container connecting means (203) are a port according to a dialysis fluid connection according to DIN EN 1283.

18. The hemodialysis machine according to any one of claims 1-7 or 17 or the hemodiafiltration machine according to claims 8-16 or 17, wherein the container (201) is a flexible bag.

19. The hemodialysis machine according to any one of claims 1-7, 17 or 18 or the hemodiafiltration machine claims 8-16, 17 or 18, wherein the sterile sampling set includes sterile container closure means (204) for closing the sampling set after sampling.

20. A method for sampling dialysis fluid of a hemodialysis machine, which has a dialysis fluid circulation that includes a source (13) and an outlet (16) for dialysis fluid, a dialysis fluid supply line (11) leading from the source (13) for dialysis fluid to a first connecting means (14), a dialysis fluid outlet line (12) leading from a second connecting means (15) to the outlet (16) for dialysis fluid, a pumping mechanism (17, 18) for circulation of dialysis fluid in the dialysis fluid circulation as well as first closure means (20) in the dialysis fluid supply line (11) and second closure means (21) in the dialysis fluid outlet line (12), wherein the first connecting means (14) and second connecting means (15) are designed so that, in carrying out a hemodialysis treatment, the connecting means are connected to the dialysis fluid chamber (4) of a hemodialyser (1) that is divided by a semipermeable membrane (2) into a dialysis fluid chamber (4) and a blood chamber (3), so that dialysis fluid flows from the source (13) through the dialysis fluid supply line (11) to the dialysis fluid chamber (4) and from the dialysis fluid chamber (4) to the outlet (16) through the dialysis fluid outlet line (12) comprising the following method steps:
closing or keeping closed the two closure means (20, 21),
connecting a sterile sampling set (200) to the first connecting means (14) or the second connecting means (15),
opening the first closure means (20) or second closure means (21), to which the sampling set (200) is connected,
operating the pump system (17, 18), so that a predefined amount of dialysis fluid flows into the sampling set (200),
closing the first closure means (20) or second closure means (21).

21. A method for sampling dialysis fluid of a hemodiafiltration machine which has a dialysis fluid circulation that includes a source (13) and an outlet (16) for dialysis fluid, a dialysis fluid supply line (11) leading from the source (13) for dialysis fluid to a first connecting means (14), a dialysis fluid outlet line (12) leading from the second connecting means (15) to the outlet (16), a substituate line (100) branching off from the dialysis fluid supply line (11) to a third connecting means (101), a pumping mechanism (17, 18) for circulation of dialysis fluid in the dialysis fluid circulation as well as first closure means (20) in the dialysis fluid supply line (11) and second closure means (21) in the dialysis fluid outlet line (12) and fifth closure means (103) in the substituate line (100), wherein the first connecting means (14) and the second connecting means (15) are designed so that, in carrying out a hemodiafiltration treatment the connecting means are connected to the dialysis fluid chamber (4) of a hemodialyser (1) that is divided by a semipermeable membrane (2) into a dialysis fluid chamber (4) and a blood chamber (3), so that dialysis fluid flows from the source (13) through the dialysis fluid supply line (11) to the dialysis fluid chamber (4), and from the dialysis fluid chamber (4) to the outlet (16) through the dialysis fluid outlet line (12), and the third connecting means (101) are designed so that in carrying out a hemodiafiltration treatment, dialysis fluid is supplied as substituate to a patient's blood through a line (5) connected to the third connecting means (101), comprising the following method steps:
closing or keeping closed the first closure means (20) and second closure means (21) or shorting the first connecting means (14) and the second connecting means (15),
closing or keeping closed the fifth closure means (103),
connecting a sterile sampling set (200) to the third connecting means (16),
opening the fifth closure means (103),
operating the pump system (17, 18), so that a predefined amount of dialysis fluid flows into the sampling set (200),
closing the fifth closure means (103).

## Revendications

1. Appareil d'hémodialyse (10) comportant un circuit de liquide de dialyse qui présente une source (13) et une évacuation (16) pour du liquide de dialyse, une conduite d'alimentation en liquide de dialyse (11) allant de la source (13) de liquide de dialyse à un premier moyen de raccordement (14), une conduite d'évacuation de liquide de dialyse (12) allant d'un deuxième moyen de raccordement (15) à l'évacuation (16) de liquide de dialyse, un dispositif de pompage (17, 18) pour renvoyer du liquide de dialyse dans le circuit de liquide de dialyse et des premiers moyens de fermeture (20) dans la conduite d'alimentation en liquide de dialyse (11) et des deuxièmes moyens de fermeture (21) dans la conduite d'évacuation de liquide de dialyse (12), et en outre une unité de commande (26) pour commander le dispositif de pompage (17, 18) et les premiers et deuxièmes moyens de fermeture (20, 21) et comportant un ensemble de prélèvement d'échantillons (200) raccordé au premier ou deuxième moyen de raccordement (14, 15) avec un moyen de raccordement de récipient (203) réalisé de manière à être complémentaire aux premiers ou aux deuxièmes moyens de raccordement (14, 15), les premiers et deuxièmes moyens de raccordement (14, 15) étant conçus de manière à ce que, lors de la réalisation d'un traitement d'hémodialyse, ils puissent être raccordés à des premières et secondes tubulures de raccordement d'une chambre de liquide de dialyse (4) d'un hémodialyseur (1) divisé par une membrane semi-perméable (2) en une chambre de liquide de dialyse (4) et une chambre destinée au sang (3) et soient complémentaires aux premières et deuxièmes nous tubulures de raccordement, afin que du liquide de dialyse puisse couler depuis la source (13) par la conduite d'alimentation en liquide de dialyse (11) vers la chambre de liquide de dialyse (4) et depuis la chambre de liquide de dialyse (4) par la conduite d'évacuation de liquide de dialyse (12) vers l'évacuation (16),
l'unité de commande (26) étant conçue de manière à commander les premiers et deuxièmes moyens de fermeture (20, 21), de même que le dispositif de pompage (17, 18), dans le cadre d'un programme de commande de prélèvement d'échantillons se déroulant automatiquement, de manière à ce qu'une quantité prédéfinie de liquide de dialyse soit transportée vers l'ensemble de prélèvement d'échantillons (200) raccordé aux premiers ou deuxièmes moyens de raccordement (14, 15).

2. Appareil d'hémodialyse selon la revendication 1, dans lequel l'ensemble de prélèvement d'échantillons est stérile et est composé d'un récipient (201) et d'une section de conduite (202) allant du récipient (201) au moyen de raccordement de récipient (203).

3. Appareil d'hémodialyse selon la revendication 1 ou 2, dans lequel les premiers et deuxièmes moyens de raccordement (14, 15) sont des accouplements adaptés aux tubulures de raccordement de liquide de dialyse (4a, 4b) conformes à la DIN EN 1283.

4. Appareil d'hémodialyse selon une des revendications précédentes, dans lequel le programme de commande de prélèvement d'échantillons, pour transporter la quantité prédéfinie de liquide de dialyse, ferme d'abord ou maintient fermés les deux moyens de fermeture (20, 21) et ouvre ensuite le premier moyen de fermeture (20) et actionne le dispositif de pompage (17) pour transporter la quantité prédéfinie de liquide de dialyse dans l'ensemble de prélèvement d'échantillons raccordé au premier moyen de raccordement (14) et referme ensuite le premier moyen de fermeture (20).

5. Appareil d'hémodialyse selon une des revendications précédentes, qui présente en outre, en amont du premier moyen de fermeture (20), une conduite de dérivation (23) bifurquant de la conduite d'alimentation en liquide de dialyse (11) et qui est pourvue d'un troisième moyen de fermeture (24) et débouche dans la conduite d'évacuation de liquide de dialyse (12) en aval du deuxième moyen de fermeture (21), un quatrième moyen de fermeture (25) étant prévu dans la conduite d'évacuation de liquide de dialyse (12) en aval du point d'embouchure.

6. Appareil d'hémodialyse selon la revendication 5, dans lequel le programme de commande de prélèvement d'échantillons, pour transporter la quantité prédéfinie de liquide de dialyse, ferme d'abord ou maintient à l'état fermé les premier (20), deuxième (21) et quatrième (25) moyen de fermeture puis ouvre ou maintient ouvert le troisième moyen de fermeture (24) et ouvre le deuxième moyen de fermeture (21) et ensuite actionne le dispositif de pompage (17) pour transporter la quantité prédéfinie de liquide de dialyse dans l'ensemble de prélèvement d'échantillons raccordé au deuxième moyen de raccordement (15) et referme ensuite le deuxième moyen de fermeture (21).

7. Appareil d'hémodialyse selon une des revendications précédentes, destiné à une utilisation comme appareil d'hémodiafiltration, dans lequel le circuit de liquide de dialyse présente une conduite de substituant (100) bifurquant de la conduite d'alimentation en liquide de dialyse (11) vers un troisième moyen de raccordement (101) et des cinquièmes moyens de fermeture (103) dans la conduite de substituant (100) et dans lequel les troisièmes moyens de raccordement (101) sont conçus de manière à ce que, lors de la réalisation d'un traitement d'hémodiafiltration, du liquide de dialyse soit acheminé dans le sang d'un patient en tant que substituant par une conduite (5) raccordée au troisième moyen de raccordement (101) et dans lequel l'unité de commande (26) est conçue de manière à ce que, dans le cadre du programme de prélèvement d'échantillons se déroulant automatiquement, elle commande également les cinquièmes moyens de fermeture (103) de manière à ce que la quantité prédéfinie de liquide de dialyse soit transportée vers l'ensemble de prélèvement d'échantillons (200) raccordé aux premiers (14) ou deuxièmes (15) moyens de raccordement.

8. Appareil d'hémodiafiltration comportant un circuit de liquide de dialyse qui présente une source (13) et une évacuation (16) pour du liquide de dialyse, une conduite d'alimentation en liquide de dialyse (11) allant de la source (13) de liquide de dialyse à un premier moyen de raccordement (14), une conduite d'évacuation de liquide de dialyse (12) allant d'un deuxième moyen de raccordement (15) à l'évacuation (16) de liquide de dialyse, une conduite de substituant (100) bifurquant de la conduite d'alimentation en liquide de dialyse (11) vers un troisième moyen de raccordement (101), un dispositif de pompage (17, 18) pour renvoyer du liquide de dialyse dans le circuit de liquide de dialyse et des premiers moyens de fermeture (20) dans la conduite d'alimentation en liquide de dialyse (11) et des deuxièmes moyens de fermeture (21) dans la conduite d'évacuation de liquide de dialyse (12) et des cinquièmes moyens de fermeture (103) dans la conduite de substituant (100), et en outre une unité de commande (26) pour commander le dispositif de pompage (17, 18) et les premiers (20), deuxièmes (21) et cinquièmes (103) moyens de fermeture et comportant un ensemble de prélèvement d'échantillons (200) raccordé au troisième moyen de raccordement (101), les premiers (14) et deuxièmes (15) moyen de raccordement étant conçus de manière à ce que, lors de la réalisation d'un traitement d'hémodiafiltration, ils puissent être raccordés à la chambre de liquide de dialyse (4) d'un hémodialyseur (1) divisé par une membrane semi-perméable (2) en une chambre de liquide de dialyse (4) et une chambre destinée au sang (3), afin que du liquide de dialyse coule depuis la source (13) par la conduite d'alimentation en liquide de dialyse (11) vers la chambre de liquide de dialyse (4) et depuis la chambre de liquide de dialyse (4) par la conduite d'évacuation de liquide de dialyse (12) vers l'évacuation (16) et que les troisièmes moyens de raccordement (101) sont conçus de manière à ce que, lors de la réalisation d'un traitement d'hémodiafiltration, du liquide de dialyse puisse être acheminé par une conduite (5) raccordée au troisième moyen de raccordement (101) dans le sang d'un patient en tant que substituant,
l'unité de commande (26) étant conçue de manière à commander les premiers (20), deuxièmes (21) et cinquièmes (103) moyens de fermeture, de même que le dispositif de pompage (17, 18), dans le cadre d'un programme de commande de prélèvement d'échantillons se déroulant automatiquement, de manière à ce qu'une quantité prédéfinie de liquide de dialyse soit transportée dans l'ensemble de prélèvement d'échantillons (200) raccordé aux troisièmes (101) moyens de raccordement.

9. Appareil d'hémodiafiltration selon la revendication 8, dans lequel l'ensemble de prélèvement d'échantillons est stérile, est composé d'un récipient (201) et d'une section de conduite (202) allant du récipient (201) au moyen de raccordement de récipient (203) et est raccordé par un moyen de raccordement de récipient (203) au troisième moyen de raccordement (101).

10. Appareil d'hémodiafiltration selon une des revendications 7 à 9, dans lequel les premiers (14) et deuxièmes (15) moyens de raccordement sont des accouplements adaptés aux tubulures de raccordement de liquide de dialyse (4a, 4b) conformes à la DIN EN 1283.

11. Appareil d'hémodiafiltration selon une des revendications 7 à 10, dans lequel le programme de commande de prélèvement d'échantillons, pour le transport de la quantité prédéfinie de liquide de dialyse, ferme ou maintient fermés d'abord les premier (20), deuxième (21) et cinquième (103) moyens de fermeture puis ouvre le cinquième (103) moyen de fermeture et actionne ensuite le dispositif de pompage (17) pour transporter la quantité prédéfinie de liquide de dialyse dans l'ensemble de prélèvement d'échantillons raccordé au troisième moyen de raccordement (101) puis referme le cinquième moyen de fermeture (103).

12. Appareil d'hémodiafiltration selon une des revendications 7 à 11, dans lequel le programme de commande de prélèvement d'échantillons, pour transporter la quantité prédéfinie de liquide de dialyse, ferme ou maintient à l'état fermé d'abord les premier (20), deuxième (21) et cinquième (103) moyens de fermeture puis ouvre le premier moyen de fermeture (20) et actionne le dispositif de pompage (17) pour transporter la quantité prédéfinie de liquide de dialyse dans l'ensemble de prélèvement d'échantillons (200) raccordé au premier moyen de raccordement (14) puis referme le premier moyen de fermeture (20).

13. Appareil d'hémodiafiltration selon une des revendications 7 à 12, celui à ci présentant en outre, en amont du premier moyen de fermeture (20), une conduite de dérivation (23) bifurquant de la conduite d'alimentation en liquide de dialyse (11) et qui est pourvue d'un troisième moyen de fermeture (24) et débouche dans la conduite d'évacuation de liquide de dialyse (12) en aval du deuxième moyen de fermeture (21), un quatrième moyen de fermeture (25) étant prévu dans la conduite d'évacuation de liquide de dialyse (12) en aval du point d'embouchure.

14. Appareil d'hémodiafiltration selon la revendication 13, dans lequel le programme de commande de prélèvement d'échantillons, pour transporter la quantité prédéfinie de liquide de dialyse, ferme ou maintien à l'état fermé d'abord les premier (20), deuxième (21), quatrième (25) et cinquième (103) moyens de fermeture puis ouvre ou maintient ouvert le troisième moyen de fermeture (24) et ouvre le deuxième moyen de fermeture (21) puis actionne ensuite le dispositif de pompage (17) pour transporter la quantité prédéfinie de liquide de dialyse dans l'ensemble de prélèvement d'échantillons (200) raccordé au deuxième moyen de raccordement et referme ensuite le deuxième moyen de fermeture (21).

15. Appareil d'hémodiafiltration selon une des revendications 6 à 14, dans lequel le troisième moyen de raccordement (101) peut être fermé par un couvercle et la surface intérieure venant en contact avec du liquide du troisième moyen de raccordement (101) peut être nettoyée par la conduite de substituant bifurquant (100) et une conduite de lavage (104) partant du troisième moyen de raccordement (101) dans un flux traversant.

16. Appareil d'hémodiafiltration selon la revendication 15, dans lequel la conduite de lavage (104) débouche en aval du deuxième moyen de fermeture (21) dans la conduite d'évacuation de liquide de dialyse (12) et est pourvue d'un sixième moyen de fermeture (105).

17. Appareil d'hémodialyse selon une des revendications 1 à 7 ou appareil d'hémodiafiltration selon une des revendications 8 à 16, dans lequel le moyen de raccordement de récipient (203) est un port correspondant à une tubulure de raccordement de liquide de dialyse conforme à la DIN EN 1283.

18. Appareil d'hémodialyse selon une des revendications 1 à 7 ou 17 ou appareil d'hémodiafiltration selon une des revendications 8 à 16 ou 17, dans lequel le récipient (201) est un sachet souple.

19. Appareil d'hémodialyse selon une des revendications 1 à 7, 17 ou 18 ou appareil d'hémodiafiltration selon une des revendications 8 à 16, 17 ou 18, dans lequel l'ensemble stérile de prélèvement d'échantillons comprend des moyens stériles de fermeture de récipients (204) pour fermer l'ensemble de prélèvement d'échantillons après le prélèvement d'échantillons.

20. Procédé de prélèvement d'échantillons de liquide de dialyse d'un appareil d'hémodialyse qui présente un circuit de liquide de dialyse qui comporte une source (13) et une évacuation (16) pour du liquide de dialyse, une conduite d'alimentation en liquide de dialyse (11) allant de la source (13) de liquide de dialyse à un premier moyen de raccordement (14), une conduite d'évacuation de liquide de dialyse (12) allant d'un deuxième moyen de raccordement (15) à l'évacuation (16) de liquide de dialyse, un dispositif de pompage (17, 18) pour renvoyer du liquide de dialyse dans le circuit de liquide de dialyse et des premiers moyens de fermeture (20) dans la conduite d'alimentation en liquide de dialyse (11) et des deuxièmes moyens de fermeture (21) dans la conduite d'évacuation de liquide de dialyse (12), les premiers (14) et deuxième (15) moyens de fermeture étant conçus de manière à ce que, lors de la réalisation d'un traitement d'hémodialyse, ils soient raccordés à la chambre de liquide de dialyse (4) d'un hémodialyseur (1) divisé par une membrane semi-perméable (2) en une chambre de liquide de dialyse (4) et nous nous une chambre destinée au sang (3), afin que du liquide de dialyse coule depuis la source (13) par la conduite d'alimentation en liquide de dialyse (11) vers la chambre de liquide de dialyse (4) et depuis la chambre de liquide de dialyse (4) par la conduite d'évacuation de liquide de dialyse (12) vers l'évacuation (16), comportant les étapes opératoires suivantes :
fermeture ou maintien à l'état fermé des deux moyens de fermeture (20, 21),
raccordement d'un ensemble stérile de prélèvement d'échantillons (200) au premier (14) ou deuxième (15) moyen de raccordement,
ouverture du premier (20) ou deuxième (21) moyen de raccordement auquel l'ensemble de prélèvement d'échantillons (200) est raccordé,
actionnement du dispositif de pompage (17, 18) afin qu'une quantité prédéfinie de liquide de dialyse coule dans l'ensemble de prélèvement d'échantillons (200),
fermeture du premier (20) ou deuxième (21) moyen de fermeture.

21. Procédé de prélèvement d'échantillons de liquide de dialyse d'un appareil d'hémodiafiltration qui présente un circuit de liquide de dialyse qui comporte une source (13) et une évacuation (16) pour du liquide de dialyse, une conduite d'alimentation en liquide de dialyse (11) allant de la source (13) de liquide de dialyse à un premier moyen de raccordement (14), une conduite d'évacuation de liquide de dialyse (12) allant d'un deuxième moyen de raccordement (15) à l'évacuation (16) de liquide de dialyse, une conduite de substituant (100) bifurquant de la conduite d'alimentation en liquide de dialyse (11) vers un troisième moyen de raccordement (101), un dispositif de pompage (17, 18) pour renvoyer du liquide de dialyse dans le circuit de liquide de dialyse et des premiers moyens de fermeture (20) dans la conduite d'alimentation en liquide de dialyse (11) et des deuxièmes moyens de fermeture (21) dans la conduite d'évacuation de liquide de dialyse (12) et des cinquièmes moyens de fermeture (103) dans la conduite de substituant (100), les premiers (14) et deuxièmes (15) moyens de fermeture étant conçus de manière à être raccordés, lors de la réalisation d'un traitement d'hémodiafiltration, à la chambre de liquide de dialyse (4) d'un hémodialyseur (1) divisé par une membrane semi-perméable (2) en une chambre de liquide de dialyse (4) et une chambre destinée au sang (3), afin que du liquide de dialyse coule depuis la source (13) par la conduite d'alimentation en liquide de dialyse (11) vers la chambre de liquide de dialyse (4) et depuis la chambre de liquide de dialyse (4) par la conduite d'évacuation de liquide de dialyse (12) vers l'évacuation (16) et les troisièmes moyens de raccordement (101) sont conçus de manière à ce que, lors de la réalisation d'un traitement d'hémodiafiltration, du liquide de dialyse soit acheminé par une conduite (5) raccordée au troisième moyen de raccordement (101) dans le sang d'un patient en tant que substituant, comportant les étapes opératoires suivantes :
fermeture ou maintien à l'état fermé des premiers (20) et deuxièmes (21) moyens de fermeture ou court-circuitage des premiers (14) et deuxième (15) moyens de fermeture,
fermeture ou maintien à l'état fermé des cinquièmes moyens de fermeture (103),
raccordement d'un ensemble stérile de prélèvement d'échantillons (200) au troisième moyen de raccordement (101),
ouverture du cinquième moyen de fermeture (103),
actionnement du dispositif de pompage (17, 18) afin de qu'une quantité prédéfinie de liquide de dialyse coule dans l'ensemble de prélèvement d'échantillons (200),
fermeture du cinquième moyen de fermeture (103).
